# EUROPEAN PATENT SPECIFICATION

(11) **EP 3 850 358 B1**
(45) Date of publication and mention of the grant of the patent: **07.06.2023**
(21) Application number: 19762822.5
(22) Date of filing: 09.09.2019
(51) Int. Cl.: G01N 33/34, G01N 13/00, G01N 35/04, D21H 27/00, G01N 5/02

(54) **DEVICE AND METHOD FOR CARRYING OUT CLEANSING EFFICACY TESTS ON A SAMPLE OF A SHEET MATERIAL**
VORRICHTUNG UND VERFAHREN ZUR DURCHFÜHRUNG VON REINIGUNGSWIRKSAMKEITSTESTS AN EINER PROBE EINES BOGENMATERIALS
DISPOSITIF ET PROCÉDÉ POUR EFFECTUER DES TESTS D'EFFICACITÉ DE NETTOYAGE SUR UN ÉCHANTILLON D'UN MATÉRIAU SOUS FORME DE FEUILLE

(30) Priority: 10.09.2018 IT 201800008454
(43) Date of publication of application: 21.07.2021
(73) Proprietor: Engraving Solutions S.r.L., 55100 Lucca (IT)
(72) Inventor: GELLI, Mauro, 55066 Capannori (LU) (IT)
(74) Representative: Mannucci, Michele
(86) International application number: PCT/EP2019/073961
(87) International publication number: WO 2020/053131

(56) References cited:
- US-A1- 2010 203 291
- US-A1- 2013 340 541
- Clear & Clean ET AL: "Test Methods for Cleaning Wipers and Papers Used in Clean Environments", , 1 August 2013 (2013-08-01), XP055502373, Retrieved from the Internet: URL:http://www.cleanboss.com/Prufmethoden_ engl._07.04.14.pdf [retrieved on 2018-08-27]
- WIN LABUDA: "Bewertung von Reinraum- Verbrauchsmaterial", PRODUCTRONIC, vol. 2003, no. 12, December 2003 (2003-12) , XP055594059,

## Description

### TECHNICAL FIELD

Disclosed herein are methods and devices for carrying out cleansing efficacy tests on samples of sheet material, for example samples of tissue paper.

### BACKGROUND ART

Tissue paper is used for the production of articles in sheet form for various uses. In particular, tissue paper is used for the production of toilet paper and for the production of kitchen towels, for use in domestic, health or professional environment. One of the important features of this type of product is its capacity to cleanse various types of surfaces, i.e. its capacity to remove substances with various consistencies, for example liquids or semi-liquids from a surface.

This cleansing capacity can vary as a function of the type of material to be removed, for example as a function of the greater or lesser density thereof, of whether it is water or oil based, of the presence of fats, etc. Cleansing capacity can also be a function of the features of the surface to be cleaned, for example of the material of which it is formed and/or of its physical and mechanical surface properties, such as greater or lesser roughness, greater or lesser of affinity toward water or oil based substances, or the like.

With the same characteristics of the surface to be cleaned and of the substances to be removed, the greater or lesser cleansing capacity can be dependent on various parameters and features of the tissue paper. For example, cleansing properties can vary as a function of the type of fiber used to produce the paper, of the thickness of the material, of the number of plies of which it is formed, of the type of embossing or other surface texture (for example obtained as a result of the structure of the forming fabric of the paper), of the type of production technique (wet or dry) of the paper, of the type of additives used, such as wet strength resins, super absorbent polymers added to the cellulose fibers, the number or mono- or multi-ply layers or sheets that are used, and the like. A for testing the cleansing capacity of a wiper sheet according to the preamble of claim 1 is disclosed in "Clear&Clean: Test methods for Cleaning Wipers and Papers Used in Cleaning Environments" August 1, 2013 (retrieved from URL:http:/www.cleanboss.com/prufmthoden_engl_07.04.14.pdf; XP055502373. A further device is disclosed in Win Labuda: "Bewertung von Reinraum-Verbrauchsmaterial". Productronic, vol.2003, n. 12, December 2003, XP055594059.

In order to optimize the characteristics related to cleansing capacity, it would be useful to have more efficient devices and methods for carrying out objective and repeatable tests on various samples in operating circumstances that can effectively simulate the conditions of normal use of the tissue paper or other sheet materials, including nonwovens, textiles or the like.

### SUMMARY OF THE INVENTION

According to a first aspect, a device is provided for carrying out cleansing efficacy tests of a sample of a sheet material, in particular a sheet of tissue paper or nonwoven, according to claim 1.

In particular, the information acquisition system can be specifically adapted to detect information on a residue of test substance on the test surface after a passage of the sample applied to the support. In other embodiments the information acquisition system can be adapted to acquire information on the test substance removed from the test surface. Preferably, the information acquisition system can be adapted to acquire a plurality of information, both relating to the test substance that remains on the test surface and to the loose material that is removed. The latter can be loose material that continues to adhere to the sample and/or loose material that is removed from the test surface but that does not continue to adhere to the sample.

Any substance (test substance, also referred here below as loose material) can be applied to the test surface, for example a water or oil based liquid substance, a fatty substance, a gel substance or any other substance that must be removed from the test surface by means of cleansing using the sample of sheet material. The substance can also be a solid substance. For example, substances in granules or powder form can be applied, such as sugar, table salt, icing sugar, wheat flour, maize flour, or the like. Through a relative movement between the test surface and the support to which the sample to be tested is applied the action of cleansing the surface using the sheet material is simulated. The relative movement is carried out holding the sample of sheet material to be tested in contact with the test surface, so as to clean this surface, completely or partially removing the substance applied thereto. After at least one passage of the sample of sheet material on the test surface has been carried out, information useful for determining the cleansing efficacy of the sheet material is acquired through the information acquisition system.

Such device allows easily repeatable objective tests to be carried out on various types of loose substances applied to the test surface. If this surface is interchangeable, it is also possible to carry out tests on surfaces with different characteristics, for example made of different materials. The test surface can, for example, be made of metal, wood, stone, conglomerate, plastic, various types of composite materials, leather, etc. Also, other characteristics of the test surface can vary from one interchangeable surface to another, for example roughness and/or capacity to retain and/or absorb the loose substances to be removed.

The device comprises a first weighing member positioned downstream of the test surface with respect to the direction of feed of the support with respect to the test surface and adapted to receive the sample from the support after the passage on the test surface. With the first weighing member it is possible to weigh the sample immediately after the cleansing action on the test surface, so as to determine (through the difference between the weight of the sample before the test) the quantity of loose material that continues to adhere to the sample.

In some embodiments the information acquisition system is adapted to acquire information on the surface distribution of the residue of loose material on the test surface. For example, the information acquisition system can comprise an image acquisition device of the test surface after the passage thereon of the sample applied to the support. The image acquisition device can be interfaced with a processing unit that carries out processing of the acquired images. For example, a comparison can be carried out between an image acquired after the passage of the sample and an image of the test surface acquired before the loose material is applied to the test surface. Various image processing operations can be carried out, for example in order to determine how much of the test surface continues to be contaminated by the loose material that is not removed and how this product is distributed on the test surface following the passage of the sample to be tested.

In some embodiments, the acquisition system can be adapted to detect a weight of the residue of loose material on the test surface. For example, a second weighing member can be provided and associated with the test surface, which can determine the weight of the residue of loose material.

The weighing members can comprise one or more load cells, which can be configured to detect even very small weights, such as milligram scales, capable of detecting quantities of the order of a few milligrams.

In the case of substances to be removed in granule or powder form, it would also be possible to use the sample to be tested in wet conditions, so that it has a greater cleansing power on the substances to be removed.

To retain the sample of sheet material to be tested on the support, the latter can comprise members for retaining and releasing the sample to be tested. In some embodiments the retaining members can be pneumatic, i.e., can comprise suction nozzles. These can be suitably positioned in areas of the support so as not to interfere with the action of the sample to be tested on the test surface. For example, the suction nozzles or holes can be positioned along perimeter edges of the support. In other embodiments, mechanical retaining systems can be provided, such as gripper members, magnetic clamping members (for example rods or a frame) or the like.

In some embodiments, members for the detachment or quick removal of the sample from the support can also be provided. For example, blowing members can be provided. The same suction nozzles can be connected to suction lines and to compressed air lines, with a system of suitably controlled valves, so as to reverse the pneumatic action exerted on the sample to be tested. In this way, it is, for example, possible to quickly remove the sample from the support and cause it to drop, for example, onto a weighing member without requiring the action of an operator. This can be useful to obtain easily repeatable tests in which the execution times (which can influence the result of the measurements) are not dependent on the skill of an operator.

The support, to which the sample to be tested is applied, can comprise a surface adapted to receive and retain the sample to be tested. The surface to which the sample to be tested is applied can be flat, but this is not essential. In some embodiments, the surface of the support, to which the sample to be tested is applied, is advantageously complementary to the test surface. Complementary means a surface that can move with respect to the test surface so that the sample remains in contact with the test surface during the relative movement. While in currently preferred embodiments the test surface and the surface to which the sample is applied are flat surfaces, in other embodiments the two surfaces can, for example, be portions of cylindrical surfaces with a circular section, with a substantially identical radius, one concave and the other convex.

In some embodiments, to facilitate carrying out the test, the support and the test surface are provided with a movement toward and away from each other in a direction orthogonal to the test surface. In this way it is also possible to adjust the contact pressure between the sample and the test surface.

In some embodiments, the device can comprise a third weighing member arranged close to a trailing edge of the test surface and adapted to receive loose material dragged by the sample to be tested beyond the outlet edge of the test surface. This is particularly the case when the loose material to be removed is in granular or powder form.

According to another aspect, a method for carrying out a cleansing efficacy test on a sample of a sheet material is provided, the method comprising the steps of:
distributing a loose material to be cleaned on a test surface;
bringing a sample of sheet material to be tested, applied to a support, into contact with the test surface;
moving the support and the test surface one parallel to the other with the sample in sliding contact with the test surface and removing the loose material from the test surface by means of the sample;
after the passage on the surface to be tested, releasing the sample on a first weighing member;
acquiring information on the effect of the passage of the sample on the test surface wherein the step of acquiring information includes weighing the sample by means of the first weighing member.

The information acquisition step can further comprise one or more of the following operations: acquiring information on the quantity and/or on the distribution of the residue of loose material on the test surface; acquiring information on the quantity of loose material that continues to adhere to the sample of sheet material; acquiring information on the quantity of loose material removed from the test surface but which does not continue to adhere to the sample of sheet material. Acquisition of the different information can require different configurations of the device and different information acquisition members, as will be apparent from the description below with reference to some examples of embodiments.

Further advantageous features and embodiments of the device and of the method according to the invention are indicated in the appended claims and will be described in detail below with reference to the accompanying drawings.

### BRIEF DESCRIPTION OF THE DRAWINGS

The invention will be better understood by following the description and the accompanying drawings, which illustrate an exemplifying and non-limiting embodiment of the invention. More particularly, in the drawings:
Fig. 1 shows a schematic side view of a test device in a first embodiment;
Fig. 2 shows a plan view according to the line II-II of Fig. 1;
Fig.3 shows a schematic section according to the line III-III of Fig.1;
Fig. 3A shows an enlargement of the detail indicated with A in Fig. 3;
Fig.4 shows a side view and partial section of a device in a different embodiment.

### DETAILED DESCRIPTION OF EMBODIMENTS

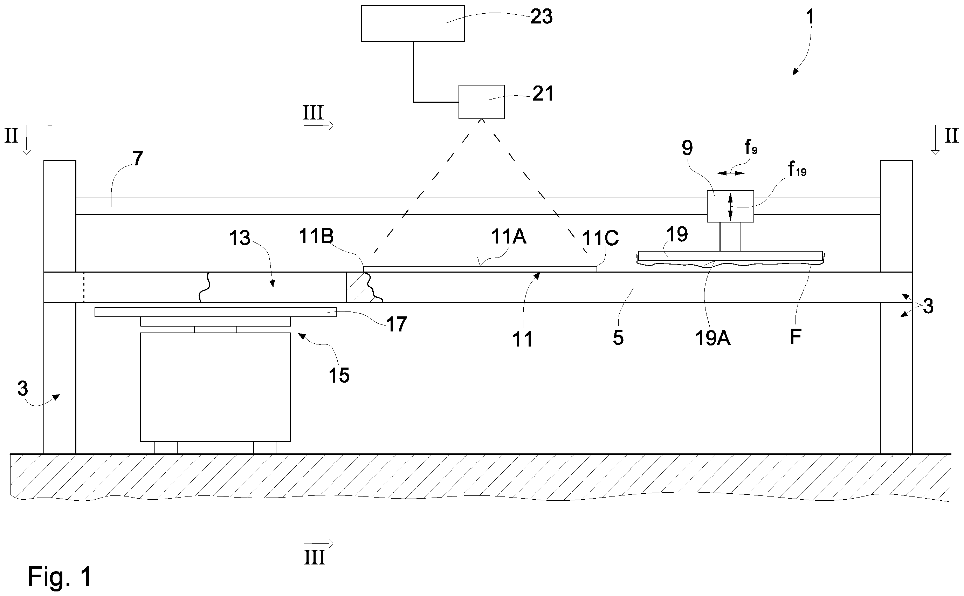

Figs.1, 2 and 3 illustrate a test device 1 in a first embodiment. The test device 1 comprises a load-bearing structure 3 comprising a table 5. The load bearing structure 3 supports, moreover, a guide system 7 on which a guide 9 slides according to a direction represented by double arrow f9. The movement according to f9 can be controlled by any kind of actuator, not shown. For example, an electronically controlled electric motor can be provided, which transmits motion to the slide 9 by means of a belt, a threaded bar, or another suitable mechanical drive.

A test plane 11 with a test surface 11A is arranged on the table. The test plane 11 can be interchangeable. For example, test planes 11 made of different materials and/or with test surfaces 11A having different textures, for example smooth or rough, shiny or opaque, etc., can be provided.

At the side of the test plane 11 the table 5 can have an opening 13, under which a first weighing member, indicated as a whole with 15, can be arranged. The weighing member 15 can have a plate or tray 17 intended to hold a sample of a sheet material on which a cleansing capacity test as described below is carried out.

The slide 9 comprises a support 19 that, in the example illustrated, has the shape of a rectangular or square plate. The support 19 comprises a lower surface 19A facing the table 5. In the embodiment illustrated the lower surface 19A of the support 19 is flat like the test surface 11A, but (although currently preferred) this is not essential.

In some embodiments the support 19 can be provided with a movement toward and away from the table 5, indicated by the double arrow f19, which can, for example, be obtained by means of a piston-cylinder actuator or a linear electric motor, not shown.

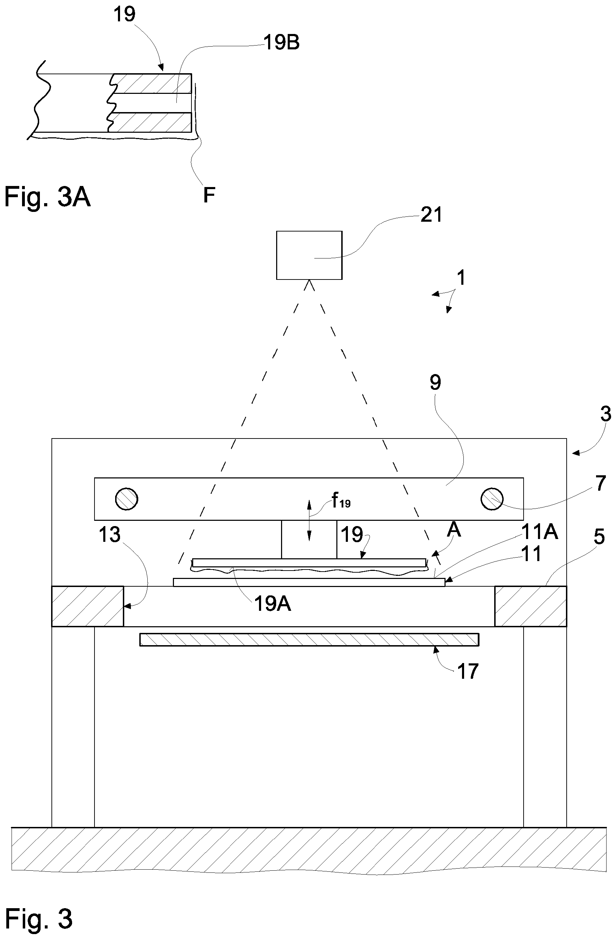

In advantageous embodiments, the support 19 comprises retaining members to retain, adhering to the lower surface 19A thereof, a sample of a sheet material to be tested, indicated schematically with F. Purely by way of example, the retaining members are represented in the enlargement of Fig. 3A as a series of suction holes 19B provided along at least some of the edges of the plate forming the support 19, for example along the edges parallel to the guides 7 and along the edge closest to the opening 13. The suction holes 19B can be connected to a suction line and optionally to a compressed air line, for example by means of suitably controlled three-way valves, such that through them a suction is exerted to retain the sample of sheet material F to be tested, wherein the suction can be interrupted promptly to release the sample of sheet material F. To facilitate and speed up detachment, jets of compressed air through the same holes 19B can be used. In other embodiments, suction holes and separate blowing holes can be provided, for example in an alternated arrangement.

In other embodiments, not shown, the retaining members can be of mechanical type and can comprise grippers, for example. In yet other embodiments, the retaining members can be magnetic or electromagnetic elements. For example, the support 19 can be provided with permanent magnets or electromagnets, to which perimeter rods or a perimeter frame can be fastened, which retain the edges of the sample of sheet material F. By using electromagnets, it is possible also to control release of the sheet material F automatically. For example, there can be provided rods mounted elastically along at least some of the edges of the support 19, on which the magnetic forces generated by electromagnets carried by the support 19 can act. By energizing the electromagnets, the rods overcome an elastic force and press against the edges of the support 19, causing the sample of sheet material F to adhere. When the electromagnets are deactivated, the rods are taken to the opening position by elastic forces, for example by means of springs. The sample of sheet material is thus released. Also, in these cases detachment of the sample of sheet material can be facilitated by jets of air.

Moreover, the device 1 can comprise an information acquisition system of information related to the cleansing effect of the sample of sheet material F on the test surface 11A. In some embodiments, the acquisition system can comprise the first weighing member 15. In the illustrated embodiment the acquisition system comprises an image acquisition device 21, for example a video camera. The video camera 21 can be interfaced with a processing unit 23 (shown schematically only in Fig.1), for example a computer, a PLC, a microcontroller, or another unit that can store and process data coming from the video camera 21. The first weighing member 15 can also be interfaced with the processing unit 23.

The video camera or other image acquisition device 21 is arranged so as to be able to acquire images of the test surface 11A for the purposes described below.

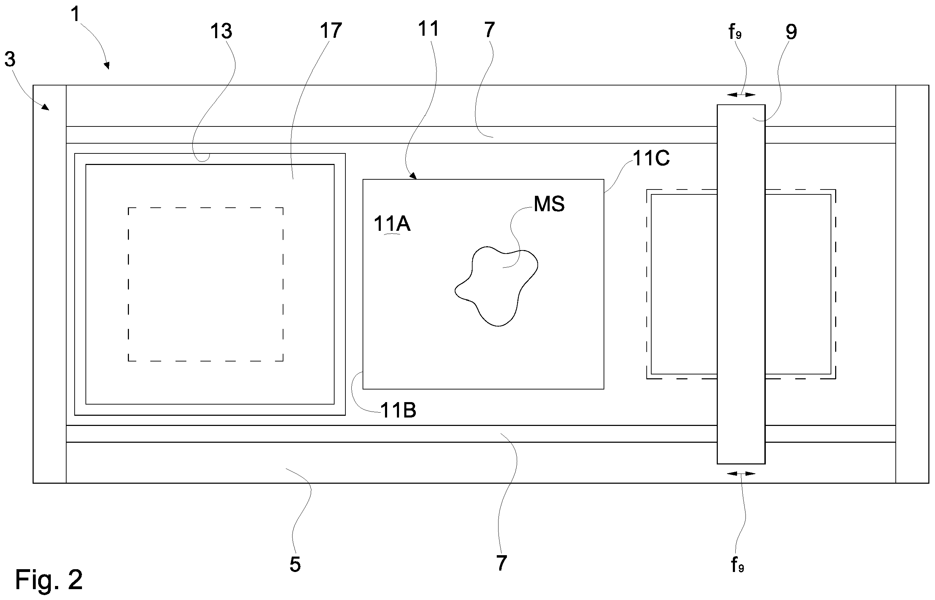

The device 1 described above can be used to carry out a cleansing capacity test on a sample of sheet material F as follows. When the support 19 is in the position illustrated in Figs. 1 and 2, the sample of sheet material F to be tested is applied to the lower surface 19A of the support 19. Subsequently, the support 19 is lowered according to the arrow f19 to take its lower surface 19A and hence the sample of sheet material F to approximately the height of the test surface 11A.

It is possible to position the support 19 so as to generate a desired pressure between the sample and the test surface 11A. Alternatively, a distance between test surface 11A and lower surface 19A less than or equal to the thickness of the sample of sheet material F can be established. In this way, it is possible to measure with constant pressure samples of sheet material F that have thicknesses that differ greatly from one another. Thickness of the sample of sheet material F means the height of the section along any linear direction of the sheet material.

A loose material MS (Fig.2) can be placed on the test surface 11A in a predetermined quantity and/or distribution. The loose material MS can, for example, be a liquid material. The loose material MS can be different for different tests. For example, in one test the loose material can simply be water, or an edible oil (vegetable oil, olive oil) or another oily or fatty substance, for example butter or margarine, or other material that simulates the consistency and the characteristics of density and surface adhesion of the oil or of other substances typically used in a domestic environment.

When the loose material MS has been applied to the test surface 11A and the support 19 has been lowered, the slide 9 can be made to translate (from right to left in Figs. 1 and 2), so as to pass the sample of sheet material F over the test surface 11A, simulating the cleaning action that is commonly carried out by the user to remove residue of material on a surface to be cleaned.

In another configuration, not shown, it is possible also to move the support 19 in a direction orthogonal and coplanar to the direction f9 so as to be able to carry out a circular, elliptical and generally curved movement of the support 19. For this purpose, the support 19 can be supported by a system of cross slides, i.e., movable on two guide systems orthogonal to each other to move the support 19 in two directions parallel to the test surface 11A and orthogonal to each other. The movement along the two directions can be numerically controlled with a numerical control according to two translation axes. A control unit can impart movements along one and/or the other of the two numerically controlled axes to generate suitable trajectories of the support 19.

The travel of the support 19 and of the slide 9 ends at the opening 13. Here the retaining members provided on the support 19 can be deactivated to cause the sample of sheet material F to drop onto the plate or tray 17 of the first weighing member 15. Weighing of the sample of sheet material F allows the quantity of loose material MS that continues to adhere to the sample to be detected. Weighing carried out immediately after the passage of the sample of sheet material F on the test surface 11A allows a precise measurement, not affected (or only negligibly affected) by phenomena of evaporation of the loose material MS collected.

The image acquisition device 21 can acquire one or more images of the test surface 11A, immediately after the passage of the sample of sheet material F. Also in this case, acquisition of the information is carried out immediately after the passage of the sample of sheet material F to be tested, so as to acquire images of the residue of loose material MS before relevant effects of evaporation, or redistribution of the liquid or semi-liquid substances, occur on the test surface 11A, for example due to the surface tension of the liquid substances. It would also be possible to acquire several images at a short distance from one another, or videos of a few seconds to evaluate the residue of loose material MS at a later time.

The information collected by the weighing member 15 and by the image acquisition device 21 provides data suitable to determine the cleansing characteristics of the sample of sheet material F tested.

Although in Figs. 1 to 3 the surfaces 11A and 19A are flat, it must be understood that this is not binding. In fact, the surfaces 11A and 19A could be mutually complementary surfaces, i.e. adapted to allow sliding of the former on the latter with a surface contact. For example, the surfaces 11A and 19A could be cylindrical, one concave and the other convex, and provided with a relative translation movement, which in this case can, for example, be obtained by means of guides that are curved instead of rectilinear like the guides 7.

In yet further embodiments, the support 19 can be configured to obtain a contact between sample of sheet material F and test surface 11A on a more limited area, for example on a line. In this case, the support 19 could have a cylindrical surface 19A that co-acts with a flat test surface 11A.

While in the description above the support 19 is lowered (arrow f19) before the start of the horizontal travel (arrow f9) of the slide or carriage 9 toward the test surface, it must be understood that this is not essential. In some embodiments, the lowering movement can be carried out when the horizontal travel has already started, for example immediately before the support 19 reaches the inlet edge (indicated with 11C in Figs.1 and 2) of the test surface 11A. It would also be possible to take the support 19 over the test plane 11 before lowering it and carrying out the lowering movement only after reaching this position. For example, it is also possible to carry out tests in which the absorption capacity of the sample of sheet material F is measured with a movement of contact and moving the sample away from the test surface 11A according to the direction 19A, without the sample of sheet material F rubbing on the test surface 11A.

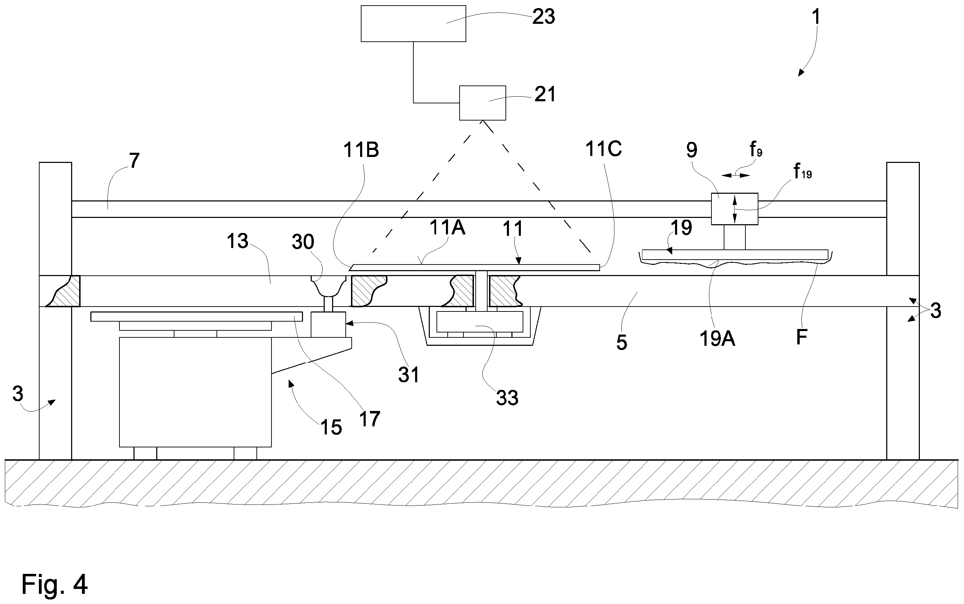

The test device 1 can be equipped with further components and devices adapted to collect further data and information. Fig.4 shows an embodiment in which a second weighing member and a third weighing member are provided. Elements identical or corresponding to those of Figs. 1 to 3 are indicated with the same reference numerals and are not described again. A further weighing member 31 supports a channel 30 arranged to collect any loose material MS that is dragged by the sample of sheet material F beyond the outlet edge 11B of the test surface 11A and that does not continue to adhere to the sample of sheet material F. Another weighing member 33 is arranged to weigh the test plane 11 so as to detect the weight of loose material MS, optionally before and/or after the passage of the sample of sheet material F to be tested. In some embodiments, only the weighing member 31 or only the weighing member 33 can be provided in combination with the first weighing member 15. The weighing members 15, 31, 33 are suitably interfaced with the processing unit 23 (Fig.1).

Although the use of an image acquisition system such as a video camera 21 is particularly advantageous, because analysis of the images of the residue of loose material on the test surface 11A provides useful information for carrying out tests on the samples of sheet material F, it would also be possible to carry out tests in which only weighted evaluations are carried out, i.e., only measurements of quantities of loose material MS weighed by the weighing members 15, 31 and 33.

When a plurality of weighing members are provided, for example the weighing members 15 and 31, it is possible to carry out tests that allow the quantity of loose material MS removed that continues to adhere to the sample of sheet material F to be distinguished from the quantity of loose material MS that is removed from the test surface 11A but does not continue to adhere to the sample of sheet material F, but drops from the outlet edge 11B of the test surface 11A.

With the test device 1 described it is possible to carry out a plurality of tests and, for example, to collect data to create a database relating to the behavior of various samples on various and different types of surfaces to be cleaned. The variables at play can be many and depend, for example, on the following parameters: type of material (paper) used, quantity of plies of which the sample of sheet material is composed, quantity of superimposed sheets, type of embossing of the sheet material, orientation of the sample of sheet material with respect to the direction (f9) of relative movement between sample and test surface 11A, type of loose material MS deposited on the test surface (viscosity, density, absorption capacity, etc.), surface finish of the test surface, nature of the test surface 11A, and the like.

As some sheet materials, and typically the paper produced with wet processes, have different physical and mechanical characteristics in machine direction (production direction in the continuous machine) and in cross direction, also the cleansing capacity can differ as a function of the orientation taken by the sample of sheet material F with respect to the direction f9. The test device 1 allows tests to be carried out with the same nature of the loose material MS, of the sheet F and of the test surface 11A, varying the orientation of the sample of sheet material F. This can be useful in attempting to obtain, during production of the sheet material, characteristics that are as homogeneous as possible, so as to make the cleansing capacity of the sheet as independent as possible from its orientation.

The device described allows particularly significant tests to be carried out, as on the one hand it is possible to automate the various operations and hence make them independent of the greater or lesser ability of the operator, eliminating spurious factors that can influence the test results. In substance, the device allows precisely repeatable and hence more easily comparable tests to be carried out. Moreover, the tests that can be carried out efficiently simulate the real use of the sheet material F, making the information collected particularly useful for understanding the effective cleansing capacity of the sample of sheet material F when used in a manner substantially equivalent to the real use. This is true for any type of surface to be cleaned and for any intended use of the material to be tested, i.e., domestic (e.g. in the kitchen), health (e.g. for the human body), or industrial (e.g. in a non-domestic working environment).

## Claims

1. A device (1) for carrying out cleansing efficacy tests of a sample of a sheet material (F), in particular a sheet of tissue paper or nonwoven, comprising in combination:
a test surface (11A) adapted to receive a predetermined dose of a test substance that must be removed from the test surface;
a support (19) adapted to retain the sample (F) of sheet material to be tested,
wherein the test surface (11A) and the support (19) are movable one with respect to the other in a direction of movement (f9) parallel to the test surface (11A), at a mutual distance so that the sample (F) applied to the support (19) slides on the test surface (11A) in contact therewith; and
an information acquisition system adapted to acquire information relating to the effect of the passage of the sample on the test surface;
**characterized by** a first weighing member (15) positioned downstream of the test surface (11A) with respect to the direction of feed of the support (19) with respect to the test surface (11A) and adapted to receive the sample (F) from the support (19) after the passage on the test surface (11A).

2. The device (1) of claim 1, wherein the information acquisition system is adapted to acquire information relating to a residue of test substance (MS) on the test surface (11A) after a passage of the sample (F) applied to the support (19).

3. The device (1) of claim 1 or 2, wherein the information acquisition system is adapted to acquire information on the surface distribution of the residue of test substance (MS) on the test surface (11A).

4. The device (1) of claim 3, wherein the information acquisition system comprises an image acquisition device (21) to acquire an image of the test surface (11A) after the passage thereon of the sample (F) applied to the support (19).

5. The device (1) of one or more of the preceding claims, wherein the information acquisition system is adapted to detect a weight of the residue of test substance (MS) on the test surface (11A).

6. The device (1) of claim 5, wherein the acquisition system comprises a second weighing member (33).

7. The device (1) of one or more of the preceding claims, wherein the support (19) comprises members (19B) for retaining and releasing the sample (F) to be tested.

8. The device (1) of one or more of the preceding claims, wherein the support (19) comprises a surface (19A) adapted to receive the sample (F) to be tested; said surface (19A) of the support (19) being complementary to the test surface (11A).

9. The device (1) of one or more of the preceding claims, wherein the support (19) and the test surface (11A) are provided with a movement toward and away from each other in a direction orthogonal to the test surface (11A).

10. The device (1) of one or more of the preceding claims, wherein the test surface (11A) is arranged on a table (5), above which guiding means (7) of the support (19) are preferably positioned; and wherein preferably the table (5) has, downstream of the test surface (11A) with respect to the direction of feed of the support (19), an opening (13), under which the first weighing member (15) is positioned, said opening having a shape and a size adapted to receive and drop therethrough the sample (F) to be tested released from the support (19).

11. The device (1) of one or more of the preceding claims, wherein the test surface (11A) is interchangeable.

12. The device (1) of one or more of the preceding claims, comprising a third weighing member (31) arranged close to a trailing edge (11B) of the test surface (11A) and adapted to receive test substance (MS) dragged by the sample (F) to be tested beyond the trailing edge (11B) of the test surface (11A).

13. A method for carrying out a cleansing efficacy test on a sample (F) of a sheet material comprising the steps of:
- distributing a test substance (MS) to be cleaned on a test surface (11A);
- bringing the sample (F) of sheet material to be tested applied to a support (19) into contact with the test surface (11A);
- moving the support (19) and the test surface (11A) one parallel to the other with the sample (F) in sliding contact with the test surface (11A) and removing the test substance (MS) from the test surface (11A) by means of the sample (F);
- after the passage on the surface to be tested (11A), releasing the sample (F) on a first weighing member (15);
- acquiring information on the effect of the passage of the sample (F) on the test surface (11A).; wherein the step of acquiring information includes weighing the sample (F) by means of the first weighing member (15).

14. The method of claim 13, wherein the step of acquiring information comprises the step of acquiring information on a residue of test substance (MS) on the test surface (11A).

15. The method of claim 14, wherein the step of acquiring information on the residue of test substance (MS) on the test surface (11A) comprises the step of acquiring at least an image of the test surface (11A) and of the residue of test substance (MS) thereon.

16. The method of claim 14 or 15, wherein the step of acquiring information on the residue of test substance (MS) on the test surface (11A) comprises the step of weighing the residue of test substance (MS) on the test surface (11A) after the passage of the sample (F).

17. The method of one or more of claims 13 to 16, wherein the step of moving the support (19) and the test surface (11A) one parallel to the other comprises the steps of:
arranging the support (19), with the sample (F) applied thereto, on a side of the test surface (11A),
translating the support (19) and the test surface (11A) one with respect to the other at a mutual distance so that the sample (F) slides in contact with the test surface (11A), causing the sample (F) to slide on the test surface (11A) from an initial position to a trailing edge (11B) of said test surface (11A).

18. The method of claim 17, further comprising the step of collecting and weighing any test substance (MS) that drops from the test surface (11A) beyond the trailing edge (11B) thereof as a result of sliding of the sample (F) on the test surface (11A).

19. The method of claim 18, comprising the step of determining the quantity of test substance (MS) that continues to adhere to the sample (F) and the quantity of test substance (MS) removed from the sample (F) that does not continue to adhere to the sample.

## Patentansprüche

1. Vorrichtung (1) zur Durchführung von Reinigungswirkungstests einer Probe eines Blattmaterials (F), insbesondere eines Blattes aus Tissue-Papier oder Vliesstoff, umfassend in Kombination:
eine Testoberfläche (11A), die zur Aufnahme einer vorbestimmten Dosis einer Testsubstanz geeignet ist, die von der Testoberfläche entfernt werden muss;
einen Träger (19), der dazu geeignet ist, die Probe (F) des zu testenden Blattmaterials zu halten, wobei die Testoberfläche (11A) und der Träger (19) in Bezug aufeinander in einer Bewegungsrichtung f19) parallel zur Testoberfläche (11A) in einem gegenseitigen Abstand beweglich sind, so dass die auf den Träger (19) aufgebrachte Probe (F) auf der Testoberfläche (11A) in Kontakt mit dieser gleitet; und
ein Informationserfassungssystem, das geeignet ist, Informationen über die Wirkung des Passierens der Probe auf der Testoberfläche zu erfassen;
**gekennzeichnet durch** ein erstes Wägeelement (15), das in Bezug auf die Vorschubrichtung des Trägers (19) in Bezug auf die Testoberfläche (11A) stromabwärts der Testoberfläche (11A) positioniert ist und die Probe (F) nach dem Passieren auf der Testoberfläche (11A) vom Träger (19) aufnehmen kann.

2. Vorrichtung (1) nach Anspruch 1, wobei das Informationserfassungssystem so ausgelegt ist, dass es Informationen bezüglich eines Rückstandes der Testsubstanz (MS) auf der Testoberfläche (11A) nach einem Passieren der auf den Träger (19) aufgebrachten Probe (F) erfasst.

3. Vorrichtung (1) nach Anspruch 1 oder 2, wobei das Informationserfassungssystem so ausgelegt ist, dass es Informationen über die Oberflächenverteilung des Rückstands der Testsubstanz (MS) auf der Testoberfläche (11A) erfasst.

4. Vorrichtung (1) nach Anspruch 3, wobei das Informationserfassungssystem eine Bilderfassungsvorrichtung (21) umfasst, um ein Bild der Testoberfläche (11A) nach dem Passieren der auf den Träger (19) aufgebrachten Probe (F) darauf zu erfassen.

5. Vorrichtung (1) nach einem oder mehreren der vorangehenden Ansprüche, wobei das Informationserfassungssystem so ausgelegt ist, dass es ein Gewicht des Rückstands der Testsubstanz (MS) auf der Testoberfläche (11A) erfasst.

6. Vorrichtung (1) nach Anspruch 5, wobei das Erfassungssystem ein zweites Wägeelement (33) umfasst.

7. Vorrichtung (1) nach einem oder mehreren der vorhergehenden Ansprüche, wobei der Träger (19) Elemente (19B) zum Festhalten und Freigeben der zu testenden Probe (F) aufweist.

8. Vorrichtung (1) nach einem oder mehreren der vorhergehenden Ansprüche, wobei der Träger (19) eine Oberfläche (19A) aufweist, die geeignet ist, die zu testende Probe (F) aufzunehmen, wobei die Oberfläche (19A) des Trägers (19) komplementär zur Testoberfläche (11A) ist.

9. Vorrichtung (1) nach einem oder mehreren der vorhergehenden Ansprüche, wobei der Träger (19) und die Testoberfläche (11A) mit einer Bewegung aufeinander zu und voneinander weg in einer Richtung senkrecht zur Testoberfläche (11A) bereitgestellt sind.

10. Vorrichtung (1) nach einem oder mehreren der vorhergehenden Ansprüche, wobei die Testoberfläche (11A) auf einem Tisch (5) angeordnet ist, über dem vorzugsweise Führungsmittel (7) des Trägers (19) angeordnet sind; und wobei vorzugsweise der Tisch (5) stromabwärts der Testoberfläche (11A) in Bezug auf die Vorschubrichtung des Trägers (19) eine Öffnung (13) aufweist, unter der das erste Wägeelement (15) positioniert ist, wobei die Öffnung eine Form und eine Größe aufweist, die geeignet ist, die zu testende Probe (F), die von dem Träger (19) freigegeben wird, aufzunehmen und hindurchfallen zu lassen.

11. Vorrichtung (1) nach einem oder mehreren der vorhergehenden Ansprüche, wobei die Testoberfläche (11A) austauschbar ist.

12. Vorrichtung (1) nach einem oder mehreren der vorhergehenden Ansprüche, die ein drittes Wägeelement (31) umfasst, das in der Nähe eines hinteren Randes (11B) der Testoberfläche (11A) angeordnet ist und so ausgelegt ist, dass es Testsubstanz (MS) aufnimmt, die von der zu testenden Probe (F) über den hinteren Rand (11B) der Testoberfläche (11A) hinaus geschleppt wird.

13. Verfahren zur Durchführung eines Reinigungswirkungstests an einer Probe (F) eines Blattmaterials, das die folgenden Schritte umfasst:
- Verteilen einer zu reinigenden Testsubstanz (MS) auf einer Testoberfläche (11A);
- in Kontakt bringen der auf einem Träger (19) aufgebrachten Probe (F) des zu testenden Blattmaterials mit der Testoberfläche (11A);
- Bewegen des Trägers (19) und der Testoberfläche (11A) parallel zueinander, wobei die Probe (F) in Gleitkontakt mit der Testoberfläche (11A) steht, und Entfernen der Testsubstanz (MS) von der Testoberfläche (11A) mit Hilfe der Probe (F);
- nach der Passage auf der zu testenden Oberfläche (11A), Freigabe der Probe (F) auf einem ersten Wägeelement (15);
- Erfassen von Informationen über die Wirkung des Passierens der Probe (F) auf der Testoberfläche (11A); wobei der Schritt des Erfassens von Informationen das Wiegen der Probe (F) mittels des ersten Wägeelements (15) umfasst.

14. Verfahren nach Anspruch 13, wobei der Schritt des Erfassens von Informationen den Schritt des Erfassens von Informationen über einen Rückstand der Testsubstanz (MS) auf der Testoberfläche (11A) umfasst.

15. Verfahren nach Anspruch 14, wobei der Schritt des Erfassens von Informationen über den Rückstand der Testsubstanz (MS) auf der Testoberfläche (11A) den Schritt des Erfassens mindestens eines Bildes der Testoberfläche (11A) und des Rückstands der Testsubstanz (MS) darauf umfasst.

16. Verfahren nach Anspruch 14 oder 15, wobei der Schritt des Erfassens von Informationen über den Rückstand der Testsubstanz (MS) auf der Testoberfläche (11A) den Schritt des Wiegens des Rückstands der Testsubstanz (MS) auf der Testoberfläche (11A) nach dem Passieren der Probe (F) umfasst.

17. Verfahren nach einem oder mehreren der Ansprüche 13 bis 16, wobei der Schritt des Bewegens des Trägers (19) und der Testoberfläche (11A) parallel zueinander die folgenden Schritte umfasst:
Anordnen des Trägers (19) mit der darauf angebrachten Probe (F) auf einer Seite der Testoberfläche (11A), Verschieben des Trägers (19) und der Testoberfläche (11A) in Bezug zueinander in einem gegenseitigen Abstand, so dass die Probe (F) in Kontakt mit der Testoberfläche (11A) gleitet, was bewirkt,
dass die Probe (F) auf der Testoberfläche (11A) von einer Anfangsposition zu einem hinteren Rand (11B) der Testoberfläche (11A) gleitet.

18. Verfahren nach Anspruch 17, ferner umfassend den Schritt des Auffangens und Wiegens jeder Testsubstanz (MS), die von der Testoberfläche (11A) über deren hinteren Rand (11B) hinaus als Ergebnis des Gleitens der Probe (F) auf der Testoberfläche (11A) fällt.

19. Verfahren nach Anspruch 18, umfassend den Schritt des Bestimmens der Menge an Testsubstanz (MS), die weiterhin an der Probe (F) haftet, und der Menge an Testsubstanz (MS), die von der Probe (F) entfernt wurde und nicht weiterhin an der Probe haftet.

## Revendications

1. Un dispositif (1) pour effectuer des tests d'efficacité de nettoyage d'un échantillon de matériau en feuille (F), en particulier une feuille de papier hygiénique ou un non tissé, comprenant en combinaison :
une surface de test (11A) apte à recevoir une dose prédéterminée d'une substance à tester qui doit être enlevée de la surface de test ;
un support (19) apte à retenir l'échantillon (F) de matériau en feuille à tester,
dans lequel la surface de test (11A) et le support (19) sont déplaçables l'une par rapport à l'autre dans une direction de déplacement (f9) parallèle à la surface de test (11A), à une distance l'une de l'autre telle que l'échantillon (F) appliqué sur le support (19) glisse sur la surface de test (11A) en contact avec lui ; et
un système d'acquisition d'informations apte à acquérir des informations relatives à l'effet du passage de l'échantillon sur la surface de test ;
**caractérisé par** un premier organe de pesée (15) positionné en aval de la surface de test (11A) par rapport à la direction d'avancement du support (19) par rapport à la surface de test (11A) et apte à recevoir l'échantillon (F) provenant du support (19) après le passage de la surface de test (11A).

2. Le dispositif (1) selon la revendication 1, dans lequel le système d'aquisition d'informations est apte à acquérir des informations relatives à un résidu de substance à tester (MS) sur la surface de test (11A) après un passage de l'échantillon (F) appliqué sur le support (19).

3. Le dispositif (1) selon la revendication 1 ou 2, dans lequel le système d'acquisition d'informations est apte à acquérir des informations sur la distribution de surface du résidu de la substance à tester (MS) sur la surface de test (11A).

4. Le dispositif (1) selon la revendication 3, dans lequel le système d'acquisition d'informations comprend un dispositif d'acquisition d'images (21) pour acquérir une image de la surface de test (11A) après le passage sur elle de l'échantillon (F) appliqué sur le support (9).

5. Le dispositif (1) selon l'une ou plusieurs des revendications précédentes, dans lequel le système d'acquisition d'informations est apte à détecter le poids du résidu de la substance à tester (MS) sur la surface de test (11A).

6. Le dispositif (1) selon la revendication 5, dans lequel le système d'acquisition comprend un deuxième organe de pesée (33).

7. Le dispositif (1) selon l'une ou plusieurs des revendications précédentes, dans lequel le support (19) comprend des organes (19B) pour retenir et relâcher l'échantillon (F) à tester.

8. Le dispositif (1) selon l'une ou plusieurs des revendications précédentes, dans lequel le support (19) comprend une surface (19A) apte à recevoir l'échantillon (F) à tester; ladite surface (19A) du support (19) étant complémentaire à la surface de test (11A).

9. Le dispositif (1) selon l'une ou plusieurs des revendications précédentes, dans lequel le support (19) et la surface de test (11A) sont animés d'un mouvement de rapprochement et d'éloignement l'un de l'autre dans une direction perpendiculaire à la surface de test (11A).

10. Le dispositif (1) selon l'une ou plusieurs des revendications précédentes, dans lequel la surface de test (11A) est agencée sur une table (5), au-dessous de laquelle des moyens de guidage (7) du support (19) sont de préférence positionnés ; et dans lequel de préférence la table (5) a, en aval de la surface de test (11A) par rapport à la direction d'avancement du support (19), une ouverture (13), sous laquelle le premier organe de pesée (15) est positionné, ladite ouverture ayant une forme et une taille aptes à recevoir et laisser passer l'échantillon (F) à tester relâché depuis le support (19).

11. Le dispositif (1) selon l'une ou plusieurs des revendications précédentes, dans lequel la surface de test (11A) est interchangeable.

12. Le dispositif (1) selon l'une ou plusieurs des revendications précédentes, comprenant un troisième organe de pesée (31) agencé à proximité d'un bord arrière (11B) de la surface de test (11A) et apte à recevoir la substance à tester (MS) entraînée par l'échantillon (F) à tester au-delà du bord arrière (11B) de la surface de test (11A).

13. Un procédé pour effectuer un test d'efficacité de nettoyage sur un échantillon (F) de matériau en feuille, comprenant les étapes consistant à :
- distribuer une substance à tester (MS) à nettoyer sur une surface de test (11A) :
- mettre l'échantillon (F) de matériau en feuille à tester appliqué sur un support (19) en contact avec la surface de test (11A) ;
- déplacer le support (19) et la surface de test (11A) parallèlement l'une à l'autre avec l'échantillon (F) en contact de glissage avec la surface de test (11A) et retirer la substance à tester (MS) de la surface de test (11A) au moyen de l'échantillon (F) ;
- après le passage sur la surface de test (11A), relâcher l'échantillon (F) sur le premier organe de pesée (15) ;
- acquérir des informations sur l'effet du passage de l'échantillon (F) sur la surface de test (11A) ; dans lequel l'étape d'acquisition d'informations inclut le pesage de l'échantillon (F) au moyen du premier organe de pesée (15).

14. Le procédé selon la revendication 13, dans lequel l'étape d'acquisition d'informations comprend l'étape d'acquisition d'informations sur un résidu de substance à tester (MS) sur la surface de test (11A).

15. Le procédé selon la revendication 14, dans lequel l'étape d'acquisition d'informations sur le résidu de substance à tester (MS) sur la surface de test (11A) comprend l'étape d'acquisition d'au moins une image de la surface de test (11A) et du résidu de la substance à tester (MS) qui est dessus.

16. Le procédé selon la revendication 14 ou 15, dans lequel l'étape d'acquisition d'informations sur le résidu de la substance à tester (MS) sur la surface de test (11A) comprend l'étape de pesée du résidu de substance à tester (MS) sur la surface de test (11A) après le passage de l'échantillon (F).

17. Le procédé selon l'une ou plusieurs des revendications 13 à 16, dans lequel l'étape de déplacement du support (19) et de la surface de test (11A) l'un par rapport à l'autre comprend les étapes consistant à :
agencer le support (19), avec l'échantillon (F) appliqué dessus, sur un côté de la surface de test (11A),
déplacer le support (19) et la surface de test (11A) l'un par rapport à l'autre à une distance l'un de l'autre telle que l'échantillon (F) glisse en contact avec la surface de test (11A), en faisant glisser l'échantillon (F) sur la surface de test (11A) d'une position initiale à un bord arrière (11B) de ladite surface de test (11A).

18. Le procédé selon la revendication 17, comprenant en outre l'étape de collecte et de pesée de n'importe quelle substance à tester (MS) qui tombe de la surface de test (11A) au-delà du bord arrière (11B) de celle-ci en raison d'un glissement de l'échantillon (F) sur la surface de test (11A).

19. Le procédé selon la revendication 18, comprenant l'étape consistant à déterminer la quantité de substance à tester (MS) qui continue à adhérer à l'échantillon (F) et la quantité de substance à tester (MS) retirée de l'échantillon (F) qui ne continue pas à adhérer à l'échantillon.
